# EUROPÄISCHE PATENTSCHRIFT

(11) **EP 1 163 955 B1**
(45) Veröffentlichungstag und Bekanntmachung des Hinweises auf die Patenterteilung: **17.03.2004**
(21) Anmeldenummer: 01110932.9
(22) Anmeldetag: 05.05.2001
(51) Int. Cl.: B01J 23/75, B01J 23/755, B01J 21/06, B01J 35/10, B01J 37/03, C07C 209/36, C07C 29/141

(54) **Hydrierkatalysator zur Reduktion funktioneller Gruppen und Verfahren zu seiner Herstellung**
Hydrogenation catalyst for reducing functional groups and process for its preparation
Catalyseur d'hydrogénation pour la réduction de groupes fonctionnels et son procédé de préparation

(30) Priorität: 14.06.2000 DE 10028436; 29.11.2000 DE 10059319
(43) Veröffentlichungstag der Anmeldung: 19.12.2001
(73) Patentinhaber: KataLeuna GmbH Catalysts, 06236 Leuna (DE)
(72) Erfinder: Birke, Peter, Prof. Dr., 06179 Langenbogen (DE); Geyer, Reinhard, Dr., 01620 Halle (DE); Schödel, Rainer, Dr., 06179 Teutschenthal (DE); Kraak, Peter, Dr., 04159 Leipzig (DE); Keck, Michael, 06667 Reichardtswerben (DE)
(74) Vertreter: Schrell, Andreas, Dr.

(56) Entgegenhaltungen:
- EP-A- 0 335 222
- EP-A- 0 672 452
- EP-A- 0 854 148
- EP-A- 1 033 361
- DE-A- 2 606 068
- DE-A- 2 712 909
- US-A- 3 794 588

## Beschreibung

Die Erfindung betrifft einen Katalysator, der für die Hydrierung von funktionellen Gruppen organischer Verbindungen in neutralen sauren oder basischen Medien, insbesondere für die Hydrierung von Nitrogruppen in Nitroaromaten zu den entsprechenden Aminen, für die Hydrierung von Aldehyden und Ketonen zu den entsprechenden Alkoholen, für die Hydrierung von Nitrilen zu den Aminen und für die reduktive Aminierung von Aldehyden und Ketonen eingesetzt werden kann und ein Verfahren zu seiner Herstellung.

Diese Hydrierungen werden sowohl im Festbettreaktor als auch im Batch-Reaktor ausgeführt. Am häufigsten werden technisch diese Hydrierungen in der Flüssigphase mit suspendiertem Katalysator vorgenommen, wobei die Verfahren sich durch die Reaktionstemperatur, Druck, Katalysator, Lösungsmittel und Art der Reaktionsführung unterscheiden.

Die katalytische Hydrierung von Glukose zu Sorbitol mit einem Ni-SiO₂-Al₂O₃-Katalysator wird in NL 8 102 190 offenbart. Der Einsatz von Ni-Cu-Trägerkatalysatoren für die Glukosehydrierung ist in der DD 217 996 beschrieben. Als Träger werden SiO₂, Al₂O₃ und SiO₂ Al₂O₃ verwendet. In DD 156 175 wird die Sorbitolgewinnung durch Hydrierung von Glukose in Gegenwart eines Ni-SiO₂ - Katalysators beschrieben. Entsprechend der Patentschrift SU 565 040 verläuft die Hydrierung zu Sorbitol am Raney- Ni bei einer Katalysatorenkonzentration von 5 - 6 %, Temperaturen von 110 bis 150 °C und Drücken von 40 bis 60 bar nach 1 bis 2h mit guten Ausbeuten. US 4 694 113 beschreibt einen Zweistufenprozeß für die Hydrierung von Glukose zu Sorbitol: In der ersten Stufe werden etwa 95 % der Glukose in Gegenwart eines Ni- Katalysators zu Sorbitol hydriert;. nach Abtrennung des Ni- Katalysators wird der Rest der Glukose mit einem Ru-Katalysator zu Sorbitol hydriert.

Die Hydrierung von 2,4-Dinitrotoluol in Gegenwart der Katalysatoren Pd/C, Raney-Kobalt beziehungsweise Platinschwarz wird im Patent J 551 33 33 offenbart. Ein Ni- Kieselgur-Trägerkatalysator wird für die Hydrierung von Di-Nitrobenzophenon zum Diamin in der EP 98 681 offenbart. In der DE 3 537 247A wird die Hydrierung von Di-Nitroverbindungen zu den Diaminen in Gegenwart von modifizierten Raney-Nickel-Katalysatoren beschrieben. Das bevorzugte Katalysatorensystem enthält neben Ni, Fe, Cr, Mo, W, V, Ti, Nb, Re, Ru, Zr und/oder Hf.

Die EP 0 335 222 lehrt die Anwendung von Ni-Al₂O₃/ZrO₂ - Trägerkatalysatoren für die Hydrierung von Nitrilen, Aromaten, Nitroverbindungen und Olefinen. Beansprucht wird insbesondere die gleichzeitige Fällung von Ni, Zr und Al auf Trägern bei 50 - 120 °C und bei pH 7,3 - 9, wobei als Träger Aktivkohle, Al₂O₃, SiO₂, Kieselgur unter anderem eingesetzt werden. Besonderes Augenmerk wird dabei auf eine homogene Ausfällung der Komponenten gelegt.

Ni-Al₂O₃/ZrO₂-Katalysatoren werden in der SU-PS 28 31 85 offenbart. Sie werden durch Fällen von Ni und Al₂O₃ auf ZrO₂ hergestellt. Gemäß der Lehre der US-PS 2 564 331 wird ein Ni- ZrO₂ - Katalysator durch Fällen eines Nickel- und Zirkoniumcarbonatgemisches mit anschließendem Waschen, Trocknen und Reduzieren bei 250 bis 350°C hergestellt. Der Katalysator enthält maximal 10 Masse-% ZrO₂.

In der DE-AS 1 257 753 wird ebenfalls die Fällung der unlöslichen Carbonate beschrieben. Der Fällvorgang wird durch Verdampfen von CO₂ und NH₃ aus einer Mischsalzlösung von Ammoniumzirkonylcarbonat und Nickelammincarbonat ausgelöst.

Die EP 0 672 452 offenbart Katalysatoren zur Hydrierung organischer Verbindungen, die im wesentlichen 65 bis 80 Masse-% Ni, berechnet als NiO, 10 bis 25 Masse-% SiO₂, 2 bis 10 Masse-% Zr, berechnet als ZrO₂ und 0 bis 10 Masse-% Al, berechnet als Al₂O₃, enthalten, wobei die Summe aus dem Gehalt Anwendung SiO₂ und Al₂O₃ mindestens 15 Masse-% beträgt. Die Herstellung dieser Katalysatoren erfolgt durch Zugabe einer sauren wässrigen Lösung von Ni-, Zr- und gewünschtenfalls Aluminiumverbindungen. Während der Fällung wird der pH-Wert zunächst auf 4,0 bis 6,5 abgesenkt und nachfolgend auf 7 bis 8 eingestellt. Das Fällprodukt wird getrocknet, calciniert und verformt.

Für die Hydrierung von Nitrilen zu Aminen werden bevorzugt Raney-Ni-Katalysatoren beschrieben. Die FR 2722710 offenbart einen Raney-Ni-Katalysator, der mit Elementen der 4.,5. und 6. Nebengruppe dotiert ist.

Raney-Ni und Raney-Co-Katalysatoren werden in der WO 9527695 für die reduktive Aminierung von Hydroxyaldehyden zu Hydroxyaminen offenbart.

Die Anwendung von Co-TiO₂-Trägerkatalysatoren mit Zusätzen von SiO₂ und ZrO₂ wird in der US-PS 5169821 für die Fischer-Tropsch-Synthese offenbart, wobei die Katalysatorherstellung in zwei Stufen erfolgt: Fällen des TiO₂ mit nachfolgender Calcination des Trägers und anschließender Auffällung des Co²⁺-carbonates.

Die bisher bekannten Ni-, Co- und Co-Ni-Hydrierkatalysatoren weisen den entscheidenden Nachteil auf, dass unter alkalischen und sauren Reaktionsbedingungen eine relativ schnelle Alterung der Katalysatoren eintritt. Grundlagenuntersuchungen ergaben, dass die Alterung insbesondere auf schnelles Wachstum der Ni-Kristallite zurückzuführen ist.

Der Erfindung liegt daher das technische Problem zugrunde, Nickel- und/oder Kobalt-haltige Trägerkatalysatoren bereitzustellen, die unter sauren oder basischen Reaktionsbedingungen eine höhere Lebensdauer aufweisen.

Dieses Problem wird erfindungsgemäß dadurch gelöst, dass ein Katalysator, der insbesondere für die Hydrierung funktioneller Gruppen in organischen Verbindungen, insbesondere für die Hydrierung von Nitrogruppen in Nitroaromaten zu den entsprechenden Aminen, für die Hydrierung von Aldehyden und Ketonen zu den entsprechenden Alkoholen, für die Hydrierung von Nitrilen zu den Aminen und für die reduktive Aminierung von Aldehyden und Ketonen, enthaltend Nickel und/oder Kobalt auf einem TiO₂haltigen Träger bereitgestellt wird, dadurch gekennzeichnet, dass der geträgerte Katalysator durch Fällen aus einer Lösung enthaltend Metall- und Ti⁴⁺-Salze hergestellt ist, wobei der Katalysator reduziert, bevorzugt reduziert und stabilisiert, ist, Metallkristallite mit Kristallitgrößen <120 Å, im reduzierten und passivierten Zustand einen Niund/oder Co-Gehalt von 20 bis 80 Masse-% (bezogen auf die Gesamtmasse des Katalysators), einen TiO₂-Gehalt von 10 - 80 Masse-%, einen SiO₂-Gehalt von 0 bis 20 Masse-%, einen ZrO₂-Gehalt von 0 - 60 Masse-%, einen HfO₂-Gehalt von 0 - 5 Masse-% (jeweils bezogen auf Gesamtmasse des Katalysators) aufweist und nach einer einstündigen Nachreduktion bei 100°C einen Reduktionsgrad von mindestens 55 % besitzt.

Die Erfindung sieht also die Bereitstellung eines Katalysators vor, der als katalytisch aktives Metall Nickel, Kobalt oder Nickel und Kobalt aufweist, also Nickel- und/oder Kobaltmetallhaltig ist, wobei diese Metalle oder dieses Metall auf einem Träger umfassend TiO₂ geträgert ist. Der TiO₂-Gehalt beträgt, bezogen auf die Gesamtmasse des Katalysators, 10 bis 80 Masse-%, vorzugsweise 20 bis 80 Masse-%, bezogen auf die Gesamtmasse des Katalysators (inklusiv Träger). Erfindungsgemäß ist in einer besonders bevorzugten Ausführungsform vorgesehen, dass der TiO₂-haltige Träger zusätzlich SiO₂- und/oder ZrO₂ und/oder HfO₂ aufweist. In einer besonders bevorzugten Ausführungsform kann der SiO₂-Gehalt bezogen auf die Gesamtmasse des Katalysators (das heißt einschließlich des Trägers) bis zu 20 Masse-% betragen. In einer weiteren bevorzugten Ausführungsform kann der ZrO₂-Gehalt bis zu 60 Masse-%, bezogen auf die Gesamtmasse des Katalysators, betragen. In einer weiteren bevorzugten Ausführungsform kann der HfO₂-Gehalt bis zu 5 Masse-% (bezogen auf die Gesamtmasse des Katalysators) betragen.

Der Reduktionsgrad wird nach einer einstündigen Nachreduktion des stabilisierten Katalysators bei 100°C im Wasserstoffstrom (Belastung: 1000 v/vh) bestimmt. Im Zusammenhang mit der vorliegenden Erfindung wird unter dem Begriff Reduktionsgrad der Anteil des metallischen Ni und/oder Co am Gesamt-Ni und Co-Gehalt verstanden.

In einer besonders bevorzugten Ausführungsform sieht die Erfindung vor, dass der vorgenannte Katalysator bei Metallgehalten von Nickel, Kobalt oder Nickel und Kobalt >50 Masse-% eine durchschnittliche Metallkristallitgröße von <100 Å. In einer weiteren bevorzugten Ausführungsform sieht die Erfindung vor, dass der Katalysator bei Metallgehalten von Nickel, Kobalt oder Nickel und Kobalt <50 Masse-% eine durchschnittliche Metallkristallitgröße von <60 Å aufweist.

In einer besonders bevorzugten Ausführungsform der Erfindung werden die reduzierten und nicht stabilisierten oder die reduzierten und stabilisierten Katalysatoren in ein geeignetes Medium eingetragen.

Als Medium können je nach Anwendungsfall Wasser, Alkohole, Fettamine oder andere Schutzmittel verwendet werden.

Die Katalysatoren können aber auch in reduzierter und stabilisierter Form als Pulver mit Korngrößen von 1 bis 200 µm, vorzugsweise von 1 bis 30 µm, eingesetzt werden. Die Katalysatoren können selbstverständlich auch als Formlinge ausgeführt sein.

Die erfindungsgemäßen Katalysatoren zeichnen sich gegenüber herkömmlichen Katalysatoren in vorteilhafter und überraschender Weise durch ihre verlängerte Lebensdauer bei verbesserter katalytischer Aktivität für die Zielprodukte aus, insbesondere, wenn alkalische oder saure Reaktionsbedingungen vorliegen. Offenbar werden bei der erfindungsgemäßen Herstellung Ni- und/oder Co-Phasen gebildet, die zu besonders aktiven und stabilen Katalysatoren führen. Der erfindungsgemäße Katalysator ist für die Hydrierung von funktionellen Gruppen organischer Verbindungen in neutralen, insbesondere aber auch in sauren oder basischen Medien besonders geeignet. Die Erfindung löst dieses Problem auch durch das Bereitstellen eines Verfahrens zur Herstellung eines solchen Katalysators.

Die Erfindung betrifft in einer weiteren Ausführungsform also auch ein Verfahren zur Herstellung des vorgenannten Katalysators, wobei durch Fällen aus einer Salzlösung enthaltend a) Ni²⁺ und Ti⁴⁺, b) Ni²⁺, Co²⁺ und Ti⁴⁺ oder c) Co²⁺ und Ti⁴⁺ durch Zugabe einer basischen Lösung, insbesondere einer Lösung von NaOH, NaHCO₃, Na₂CO₃ oder eines Gemisches mindestens zweier dieser Substanzen, bis zu einem pH-Wert von 7 bis 9,5 ein Fällprodukt erhalten wird, welches nach Filtration gewaschen, danach bei Temperaturen von 80°C bis 150°C getrocknet, anschließend bei Temperaturen von 250 bis 700°C calciniert, gegebenenfalls danach inertisiert und anschließend mit Wasserstoff bei Temperaturen von 300°C bis 550°C reduziert, gegebenenfalls inertisiert und abschließend stabilisiert wird oder in ein Medium eingetragen wird, wobei mindestens 20 % des TiO₂haltigen Trägers im reduzierten Katalysator durch die gemeinsame Fällung aus der Lösung enthaltend das Gemisch aus Salzen erhalten wird.

In einer besonders bevorzugten Ausführungsform der vorliegenden Erfindung kann vorgesehen sein, dass die Lösung enthaltend ein Gemisch aus Salzen, also die Salzlösung, enthaltend a) Ni²⁺ und Ti⁴⁺, b) Ni²⁺, Co²⁺ und Ti⁴⁺ oder c) Co²⁺ und Ti⁴⁺ zusätzlich Zr⁴⁺ enthält. In einer weiteren bevorzugten Ausführungsform kann vorgesehen sein, dass die Lösung enthaltend ein Gemisch aus Salzen neben den vorgenannten Komponenten auch Hf⁴⁺ enthält. In einer weiteren bevorzugten Ausführungsform wird der basischen Lösung von NaOH, NaHCO₃, Na₂CO₃ oder eines Gemisches mindestens zweier dieser Substanzen eine lösliche Si⁴⁺-Verbindung, zum Beispiel Wasserglas zugesetzt.

In einer besonders bevorzugten Form enthält die Lösung enthaltend ein Gemisch aus Salzen Anteile von TiO₂ oder TiO₂-ZrO₂-Gemischen oder TiO₂-ZrO₂-SiO₂-Gemischen, vorzugsweise in suspendierter Form, wobei im erfindungsgemäß hergestellten reduzierten Katalysator der Anteil des Trägers aus den oxidischen Zusätzen in besonders bevorzugter Ausführungsform 80 Masse-% (bezogen auf Masse des Trägers) nicht übersteigt. Maximal 80 % der Masse des Trägers stammen aus dem Zusatz von festem TiO₂, ZrO₂ beziehungsweise SiO₂. Die restlichen Anteile des Trägers stammen aus der Fällung aus den Ti⁴⁺- und Zr⁴⁺-Salzlösungen.

Die Erfindung sieht also in besonders bevorzugter Ausführungsform vor, dass die als Träger eingesetzten Substanzen wie TiO₂, ZrO₂ oder SiO₂ in der Fälllösung sowohl in fester als auch in gelöster Form eingesetzt werden können.

Die Herstellung des Fällproduktes erfolgt also durch Zugabe der genannten basischen Lösung zu der Ni²⁺- oder/und Co²⁺- und Ti⁴- oder Ti⁴⁺- und Zr⁴⁺-Salzlösung, die gegebenenfalls TiO₂ oder TiO₂-ZrO₂-Gemische oder TiO₂-ZrO₂-SiO₂-Gemische enthält, wobei diese Zugabe soweit und solange erfolgt, bis die Fällsuspension einen pH-Endwert von 7,5-9,5 erreicht. In einer weiteren bevorzugten Ausführungsform ist vorgesehen, dass der genannten basischen Lösung eine lösliche Si⁴⁺-Verbindung, zum Beispiel Wasserglas, zugesetzt wird.

Des weiteren sieht die Erfindung in einer bevorzugten Ausführungsform vor, dass die Fällung bei Temperaturen von 65°C bis 100°C vorgenommen wird. In bevorzugter Ausführung kann vorgesehen sein, dass nach Erreichen des End-pH-Wertes die erhaltene Fällsuspension zum Beispiel für 0,5 bis 4 Stunden nachgerührt wird, bevor eine Weiterbearbeitung erfolgt. Die Reduktion und Passivierung oder der Eintrag des Ni- und/oder Co -haltigen Trägerkatalysators in ein Schutzmedium kann in üblicherweise vorgenommen werden und ist in den Ausführungsbeispielen beschrieben.

Weitere vorteilhafte Ausgestaltungen der Erfindung ergeben sich aus den Unteransprüchen.

Der Gegenstand der Erfindung wird an folgenden Beispielen veranschaulicht.

### Beispiel 1 (erfindungsgemäß)

81 Wasser werden in einem mit einem Rührer versehenen, beheizbaren Fällbehälter vorgelegt und anschließend werden 1,2 kg Soda zugegeben. Nach dem Sodazusatz erfolgt das Aufheizen der Suspension auf eine Temperatur von 80 - 90°C unter Rühren. Nach dem vollständigen Lösen der Soda beginnt die Dosierung einer Lösung, die neben Nickel und Titan in Form von Nitrat beziehungsweise Oxisulfat auch festes Titanoxid enthält. Das Molverhältnis von Nickel zu TiO₂ beträgt ca. 4. Das Masseverhältnis des als Titanylsulfat in Lösung befindlichen TiO₂ zum festen TiO₂ liegt bei etwa 3. Die Fälltemperatur liegt bei ca. 85°C. Die Fällung ist beim Erreichen eines pH-Wertes von 8 - 8,5 abgeschlossen. Die Gesamtfällzeit beträgt ca. 2h. Im Anschluss an die Fällung wird die Suspension noch ca. 2h bei obigen Temperaturen nachgerührt, danach filtriert und so lange mit alkalifreiem Wasser gewaschen bis der Na₂O-Gehalt im Filterkuchen <0,3 %, bezogen auf den Glührückstand des bei 800°C getemperten Filterkuchens, liegt. Anschließend wird der Filterkuchen ca. 15h bei Temperaturen von 120 - 150°C getrocknet und bei 450°C calciniert.

Nach der Calcinierung wird das Zwischenprodukt gemahlen, tablettiert, im Wasserstoffstrom 6h bei 420°C reduziert und in einem CO₂/Stickstoffstrom mit einem Sauerstoffgehalt von 0,1 bis 1 Vol.% bei Temperaturen unter 80°C behandelt.

Der reduzierte und stabilisierte Katalysator enthält ca. 65 % Nickel und 20 % TiO₂ bezogen auf den Gesamtkatalysator. Er weist einen Nickelreduktionsgrad, ausgedrückt durch das Verhältnis von metallischem Nickel zum Gesamtnickel von ca. 80 %, auf. Die mittlere Nickelprimärteilchengröße liegt bei 59 Å.

### Beispiel 2 (erfindungsgemäß)

81 Wasser werden in einem beheizbaren Fällbehälter vorgelegt und anschließend wird eine Nickel, ZrO₂ und TiO₂ enthaltende Lösung zugegeben. Das Molverhältnis von Nickel zu ZrO₂ zu TiO₂ liegt bei 1:0,07:0,11. Das Zirkonoxid wurde sowohl in fester als auch in gelöster Form als ZrO₂ beziehungsweise Zirkonylnitratlösung zugesetzt. Das Masseverhältnis von in Lösung befindlichem zum festem Zirkon betrug bei dem Fällansatz ca. 3. Nach der Zugabe der Lösung wurde unter Rühren auf eine Temperatur von 80°C aufgeheizt und anschließend mit einer Sodalösung (150g Soda/l Lösung) bis zum pH-Wert von 8 - 8,5 gefällt. Die Fäll- und Nachrührzeit betrug ebenfalls je 2h.

Die weitere Verarbeitung des Katalysators erfolgte wie im Beispiel 1. Die Reduktion des Katalysatorvorläufers wurde 6h bei einer Temperatur von 450°C vorgenommen.

Der Fertigkatalysator hatte einen Nickelgehalt von ca. 66 %, einen ZrO₂- und einen TiO₂-Gehalt von je ca. 10 %. Der Reduktionsgrad lag bei ca. 85 %. Die mittlere Nickelprimärteilchengröße betrug 80 Ä.

### Beispiel 3 (erfindungsgemäß)

81 Wasser werden in einem mit einem Rührer versehenen, beheizbaren Fällbehälter vorgelegt und anschließend erfolgt die Dosierung einer Lösung, die Kobalt und Titan in Form von Nitrat beziehungsweise Oxysulfat enthält. Das Molverhältnis von Kobalt zu TiO₂ in der Metalllösung beträgt ca. 2. Nach Zugabe der Lösung wird auf eine Temperatur von 80°C unter Rühren aufgeheizt und anschließend nach Erreichen der Temperatur mit der Fällung begonnen. Als Fällmittel dient eine wässrige Sodalösung mit einer Na₂CO₃-Konzentration von ca. 180g/l. Die Fällzeit und Nachrührzeit bei Temperaturen von 80 - 90°C betragen je etwa 2h. Die Fällung war nach Einstellung eines pH-Wertes von 8,5 - 8,8 abgeschlossen.

Die Trocknung des Katalysators erfolgte wie im Beispiel 1 beschrieben. Die Calcinierung wurde bei Temperaturen von 350°C vorgenommen. Im Anschluss an die Calcinierung wurde das körnige Zwischenprodukt grob gemahlen und anschließend unter Zusatz von Graphit locker tablettiert. Die Reduktion im Wasserstoffstrom wurde bei 350°C vorgenommen und danach wurde der Katalysator im CO₂/Stickstoffstrom mit O₂-Gehalten von 0,1 - 0,5 Vol.% stabilisiert.

Der fertige Katalysator enthält ca. 50 % Kobalt und 35 % TiO₂. Der Reduktionsgrad lag bei ca. 60 % und die mittlere Kristallitgröße bei 115 Å.

Die reduzierten und stabilisierten Katalysatoren der erfindungsgemäßen Beispiele 1 bis 3 werden auf eine Korngröße von <63 µm gemahlen und anschließend für die Suspensionphasenhydrierung von Glucose bei 135°C und 125 bar (Beispiel 1-3) eingesetzt. Die Katalysatoren der erfindungsgemäßen Beispiele 1 und 2 werden zudem in der Nitrobenzenhydrierung katalytisch getestet.

### Beispiel 4 (erfindungsgemäß)

81 Wasser werden im Fällbehälter vorgelegt und anschließend werden ca. 1,4 kg NaHCO₃ unter Rühren zugegeben. Danach erfolgt das Aufheizen auf eine Temperatur von ca. 80°C und nach vollständigem Lösen des Bicarbonates wird mit der Fällung begonnen. Die zum Einsatz kommende Metalllösung enthält Nickel, Zirkon und Titan in Form von Nitrat, Oxinitrat und Oxisulfat. Das Molverhältnis von Nickel zu ZrO₂ zu TiO₂ in der Lösung beträgt 1 : 0,143 : 0,22. Die Fällung wird bei einer Temperatur von 80°C bis zu einem pH-Wert von 8,8 durchgeführt. Die Fäll- und Nachrührzeiten sind mit denen in den vorigen Beispielen genannten identisch. Die Fällsuspension wird anschließend filtriert und der Filterkuchen mit Reinstkondensat gewaschen. Der Filterkuchen wird danach erneut in Wasser redispergiert und die so hergestellte Slurry in einem Sprühtrockner versprüht. Das anfallende pulverförmige Trockenprodukt wird unter Verwendung von Wasser, HNO₃ und einem geeigneten Bindemittel peptisiert und anschließend zu 2 mm Vollsträngen extrudiert. Nach Trocknung und Calcinierung bei Temperaturen von 130°C beziehungsweise 400°C erfolgt die Reduktion der Formlinge bei einer Temperatur von 420°C im Wasserstoffstrom. Die Stabilisierung wird wie im Beispiel 1 beschrieben vorgenommen.

Der fertige Katalysator hat einen Gehalt an Nickel von ca. 55 %, der ZrO₂ und TiO₂-Gehalt lag bei je ca. 17 %. Der Hafniumgehalt (berechnet als HfO₂) beträgt ca. 0,5 %. Der Reduktionsgrad ist ca. 80 %, die mittlere Kristallitgröße 52 Å und die Kantenfestigkeit der Extrudate ca. 8 N/mm. Der Hafniumgehalt resultiert aus den Verunreinigungen der Zr⁴⁺-Salzlösung.

Der Katalysator wird für die Hydrierung von Glucose im Festbettverfahren bei einer Temperatur von 105 - 120°C und einem Druck von 220 bar eingesetzt. Verwendet wurde eine 40 %ige Glucoselösung.

### Beispiel 5 (erfindungsgemäß)

Wie im Beispiel 1 beschrieben, wird Wasser im Fällbehälter vorgelegt und danach Soda unter Rühren zugesetzt. Nach Lösen der Soda wird festes TiO₂ mit einer mittleren Korngröße von 7 µm in die Lösung gegeben und anschließend auf eine Temperatur von 90 - 95°C aufgeheizt. Die Fällung wird unter Einsatz einer vereinigten Nickel-, Kobaltnitrat- und Titanoxisulfatlösung bis zu einem pH-Wert von 8,5 durchgeführt. Die Fäll- und Nachrührzeit beträgt je 1,5h. Das Molverhältnis von Nickel zu Kobalt zu TiO₂ liegt in der Fälllösung bei 1 : 0,33 : 0,65. Das Masseverhältnis zwischen festem und gelöstem TiO₂ ist 1.

Nach Abschluss der Fällung wird, wie schon beschrieben, filtriert, gewaschen und der Filterkuchen im Wasser redispergiert und versprüht. Die mittlere Korngröße des versprühten Materials liegt bei 10 µm. Das Trockenprodukt wird bei 380°C reduziert. Nach Abschluss der Reduktion wird im Inertgasstrom abgekühlt und danach das pyrophore, reduzierte Katalysatorpulver unter Luftausschluss in ein handelsübliches gehärtetes Talgfettamin (Distearylamin) eingetragen.

Der reduzierte (nicht eingetragene) Katalysator weist eine Zusammensetzung von 40 % Nickel, 13 % Kobalt und 33 % TiO₂ auf. Der Reduktionsgrad liegt bei 85 %, die mittlere Metallkristallitgröße bei 93 Å.

Der Katalysator wurde für die Hydrierung von Fettsäurenitrilen zu primären Aminen in der Suspensionsphase getestet.

### Beispiel 6 Vergleichsbeispiel

305,76 g Ni (NO₃)₂ 6 H₂O und 29,52 g Al (NO₃)₃ 9H₂O werden in 1760 ml destilliertem Wasser und 2,32 g Zirkoncarbonat in 9 ml HNO₃ (56 Masse-%) gelöst. Beide Lösungen werden vereinigt und auf 101°C erhitzt. Diese Mischsalzlösung wird innerhalb von 3 Minuten gleichmäßig zu einer 100°C heißen und intensiv gerührten Sodalösung gegeben, die aus 147,04 g Soda und 1416 ml destilliertem Wasser hergestellt worden ist. In die frisch gefällte Suspension werden 27,76 g Kieselgur eingerührt und die dabei entstehende Mischung wird noch weitere 3 Minuten gerührt. Das Fällprodukt wird anschließend filtriert und mit heißem Wasser gewaschen, bis der Alkaligehalt des Waschwassers ca. 20 mg Na₂O/l beträgt. Der auf diese Weise erhaltene Filterkuchen wird in 70°C heißem Wasser suspendiert (Mengenverhältnis Filterkuchen zu Wasser = 1:1), 60 Minuten gerührt und nachfolgend erneut filtriert. Der hierbei entstehende Filterkuchen wird zu zylinderförmigen Formkörpern (Durchmesser 5 mm, Länge 8 bis 10 mm) extrudiert und anschließend bei steigender Temperatur (50 bis 60°C) mit Luft auf einen Restgehalt an Wasser <10 Masse-%, bezogen auf getrocknete Masse, getrocknet. Das getrocknete Material wird im Wasserstoffstrom nach vorheriger Inertisierung im Stickstoffstrom (1000 v/vh, 30 Minuten) mit einer Belastung von 400 v/vh und einer Aufheizzeit von 5°C/min auf 470°C hochgeheizt und über einen Zeitraum von 4h bei dieser Temperatur reduziert. Die Stabilisierung wurde wie in den erfindungsgemäßen Beispielen vorgenommen.

Die Katalysatoren wurden wie folgt katalytisch charakterisiert:
A) Hydrierung von Glucose mit einem 0,5 l Rührautoklav und einer Wasserstoffverbrauchsmessung bei konstantem Druck:

| | |
|---|---|
| Reaktionsgemisch | 120 g Glucose, 90 g Wasser und 1,2 g Katalysator (Korngröße <63 µm) |
| Druck | 125 bar |
| Reaktionstemperatur | 135°C |
| Rührgeschwindigkeit | 2000 Umdrehungen / Minute |

Als Aktivitätsmaß dient die Reaktionszeit, die erforderlich ist, um 98,5 % der Glucose zu hydrieren.
B) Hydrierung von Nitrobenzol zu Anilin im 0,5 1 Autoklav mit Wasserstoffverbrauchsmessung bei konstantem Reaktionsdruck:

| | |
|---|---|
| Reaktionsgemisch | 80 g Nitrobenzol, 40 g Wasser und 0,25 g Katalysator (Korngröße <63 µm) |
| Reaktionsdruck | 25 bar |
| Reaktionstemperatur | 120°C |
| Rührgeschwindigkeit | 2000 Umdrehungen / Minute |

Als Maß für die Hydrieraktivität diente die Zeit, in der 100 % des Nitrobenzols umgesetzt worden sind.
C) Hydrierung von Glucose zu Sorbitol im Festbettverfahren

| | |
|---|---|
| Einsatzsubstrat | 40 %ige wässrige Glucoselösung |
| Reaktionsdruck (bar) | 220 |
| Reaktionstemperatur (°C) | variabel bis zum Erreichen eines Mindestumsatzes von 99,8 % |
| Durchsatz Glucoselsg. (l/h) = | 0,7 |
| Belastung (v/vh) | 1 |

Vor Beginn der Hydrierung werden die Katalysatoren 4h bei 180°C im Wasserstoffstrom aktiviert. Als Aktivitäts- und Selektivitätsmaß dient die Reaktionstemperatur, die zum Erreichen eines Umsatzes von 99,8 % eingestellt werden muss sowie die Sorbitolausbeute.
D) Hydrierung von Oleylnitril zu Oleylamin
Batchverfahren

| | |
|---|---|
| Reaktionsgemisch | 150 g Oleylnitril, 33 g Ammoniak und 2,5 g Katalysator (in Fett eingetragen); Einwaage: ohne Fett |
| Reaktionstemperatur | 140°C |
| Reaktionsdruck | 75 bar |
| Rührgeschwindigkeit | 2100 Umdrehungen / Minute |
| Hydrierzeit | 130 Minuten |

Die Stabilität der Katalysatoren wurde durch die durchschnittliche Metallkristallitgröße charakterisiert, die sich nach einer 100-stündigen Behandlung im Reaktionsgemisch nach der Hydrierung des Nitrobenzols unter den Druck- und Temperaturbedingungen der Hydrierung einstellt.

Die mittlere Metallkristallgröße wurde mit einem Meßplatz der Firma Rich. Seifart & Co. Freiberger-Präzisionsmechanik GmbH bestimmt, indem für Nickel und für kubisch kristallisiertes Co die Streukurvenausschnitte senkrecht zur (200) - Netzebene beziehungsweise für hexagonal kristallisiertes Co senkrecht zur (101) - Netzebene aus der Interferenzlinienverbreiterung unter folgenden Bedingungen aufgenommen worden sind.

| | |
|---|---|
| Generatordaten | 34 kV/20mA |
| Goniometer | HZG4 |
| Strahlung | Cu-Kα |
| Filter | C - Monochromator |
| Winkelbereich | 2Θ = 41° - 49° |
| Schrittweite | ΔΘ = 0,05° |
| Zählzeit | 20 s |

Die Katalysatoren werden durch die Daten der folgenden Tabellen charakterisiert:

**Tabelle 1**

| Ergebnisse der katalytischen Prüfung in der Nitrobenzol-und Glucosehydrierung (Suspensionsverfahren) | | | | | |
|---|---|---|---|---|---|
| Katalysator | Hydrierzeit für Nitrobenzol in min (Umsatz: 100 %) | Anilinausbeute in % | Hydrierzeit für Glucose in min (Umsatz: 98,5 %) | mittlere Ni-Kristallitgröße in Å (vor der Reaktion) | mittlere Ni-Kristallitgröße in Å (nach dem Stabilitätstest) |
| Beispiel 1 | 90 | 99,3 | 38 | 59 | 64 |
| Beispiel 2 | 94 | 99,2 | 40 | 80 | 88 |
| Beispiel 3 | - | - | 37 | - | - |
| Beispiel 6 (Vergleichsbeispiel) | 129 | 98,7 | 50 | 72 | 107 |

**Tabelle 2**

| Ergebnisse der katalytischen Prüfung in der Glucosehydrierung (Festbettverfahren) | | |
|---|---|---|
| Katalysator | Temperatur für das Erreichen des Umsatzes von 99,8 % | Sorbitolausbeute¹⁾ in % |
| Beispiel 4 | 105°C | 98,6 |
| Beispiel 6 (Vergleichsbeispiel) | 114°C | 96,1 |

| | | |
|---|---|---|
| 1) nach 100 h Reaktionszeit | | |

**Tabelle 3**

| Ergebnisse der katalytischen Prüfung bei der Hydrierung von Oleylnitril zu Oleylamin | | |
|---|---|---|
| Katalysator | Umsatz in % | Ausbeute an Oleylamin (primär) in % |
| Beispiel 5 | 99,1 | 98,8 |
| Beispiel 6 (Vergleichsbeispiel) | 98,0 | 96,4 |

Die Vorzüge der erfindungsgemäßen Katalysatoren werden durch die hohen katalytischen Aktivitäten und hohen Stabilitäten ausgewiesen. Die besseren Stabilitäten werden durch die geringen Zunahmen der Metallkristallitgröße belegt.

## Patentansprüche

1. Katalysator, insbesondere für die Hydrierung von funktionellen Gruppen organischer Verbindungen, enthaltend Nickel und/oder Kobalt auf einem TiO₂haltigen Träger **dadurch gekennzeichnet, dass** der geträgerte Katalysator durch Fällen aus einer Lösung enthaltend Katalysatormetall- und Ti⁴⁺-Salze hergestellt ist, reduziert ist, Metallkristallite mit Kristallgrößen von <120 Å aufweist, im reduzierten und passivierten Zustand einen GesamtGehalt von 20 bis 80 Masse-% (bezogen auf die Gesamtmasse des Katalysators) an Nickel, Kobalt oder Nickel und Kobalt besitzt, einen TiO₂-Gehalt von 10 bis 80 Masse-% besitzt und nach einer einstündigen Nachreduktion bei 100°C einen Reduktionsgrad von mindestens 55 % besitzt.

2. Katalysator nach Anspruch 1, wobei der TiO₂haltige Träger einen SiO₂-Gehalt von maximal 20 Masse-% (bezogen auf Gesamtmasse des Katalysators) aufweist.

3. Katalysator nach Anspruch 1 oder 2, wobei der TiO₂-haltige Träger einen ZrO₂-Gehalt von maximal 60 Masse-% (bezogen auf Gesamtmasse des Katalysators) aufweist.

4. Katalysator nach einem der Ansprüche 1 bis 3, wobei der TiO₂-haltige Träger einen HfO₂-Gehalt von maximal 5 Masse-% (bezogen auf die Gesamtmasse des Katalysators) aufweist.

5. Katalysator nach Anspruch 1, wobei der reduzierte Katalysator stabilisiert ist.

6. Katalysator nach einem der vorhergehenden Ansprüche, wobei der Katalysator einen Gesamtmetallgehalt von Kobalt und/oder Nickel >50 Masse-% und eine durchschnittliche Metallkristallitgröße von <100 Å aufweist.

7. Katalysator nach einem der Ansprüche 1 bis 5, wobei der Katalysator einen Metallgehalt von Kobalt und Nickel <50 Masse-% und eine durchschnittliche Metallkristallitgröße von <60 Å aufweist.

8. Katalysator nach einem der Ansprüche 2 bis 4, wobei der Katalysator in reduzierter und stabilisierter Form als Pulver mit Korngrößen von 1 bis 200 µm vorliegt.

9. Katalysator nach Anspruch 5, wobei der Katalysator als Pulver mit Korngrößen von 1 bis 30 µm vorliegt.

10. Katalysator nach einem der Ansprüche 1 bis 8, wobei der Katalysator in Form von Extrudaten, Kugeln oder Tabletten vorliegt.

11. Katalysator nach einem der vorhergehenden Ansprüche, wobei der Katalysator in einem geeigneten Medium ausgewählt aus der Gruppe bestehend aus Wasser, Alkoholen, Fettaminen oder anderen Schutzmitteln vorliegt.

12. Verfahren zur Herstellung eines Nickelund/oder Kobalt-haltigen Katalysators auf einem Träger, insbesondere für die Hydrierung von funktionellen Gruppen organischer Verbindungen, insbesondere nach einem der Ansprüche 1 bis 11, wobei durch Fällen aus einer Lösung enthaltend ein Gemisch aus Katalysatormetall- und Ti⁴⁺-Salzen ausgewählt aus der Gruppe bestehend aus a) Ni²⁺ und Ti⁴⁺, b) Ni²⁺ und Co²⁺ und Ti⁴⁺, und c) Co²⁺ und Ti⁴⁺ mit einer basischen Lösung bis zu einem pH-Wert von 7 bis 9,5 ein Fällprodukt erhalten wird, welches nach Filtration gewaschen, danach bei Temperaturen von 80°C bis 150°C getrocknet, bei Temperaturen von 250 bis 700°C calciniert, und mit Wasserstoff bei Temperaturen von 300°C bis 550°C reduziert wird, wobei mindestens 20 % des TiO₂-haltigen Trägers im reduzierten Katalysator durch die gemeinsame Fällung aus der Lösung enthaltend das Gemisch aus Salzen erhalten wird.

13. Verfahren nach Anspruch 12, wobei die basische Lösung eine Lösung von NaOH, NaHCO₃ oder Na₂CO₃ oder eines Gemisches mindestens zweier dieser Substanzen ist.

14. Verfahren nach Anspruch 12 oder 13, wobei der Katalysator nach dem Calcinieren inertisiert wird.

15. Verfahren nach einem der Ansprüche 12 bis 14, wobei der Katalysator nach der Reduktion inertisiert wird.

16. Verfahren nach einem der Ansprüche 14 oder 15, wobei der reduzierte und gegebenenfalls inertisierte Katalysator im Anschluss an die Reduktion beziehungsweise Inertisierung stabilisiert wird.

17. Verfahren nach einem der Ansprüche 14 bis 16, wobei der Katalysator nach der Reduktion und gegebenenfalls erfolgten inertisierung direkt in ein Medium eingetragen wird.

18. Verfahren nach einem der Ansprüche 12 bis 17, wobei die Lösung enthaltend ein Gemisch aus Salzen Zr⁴⁺ enthält.

19. Verfahren nach einem der Ansprüche 12 bis 18, wobei die Lösung enthaltend ein Gemisch aus Salzen Anteile von TiO₂ oder TiO₂-ZrO₂-Gemischen oder TiO₂-ZrO₂-SiO₂-Gemischen enthält, vorzugsweise in suspendierter Form, wobei der Anteil des TiO₂haltigen Trägers im reduzierten Katalysator aus den oxidischen Zusätzen maximal 80 % beträgt.

20. Verfahren nach einem der Ansprüche 12 bis 19, wobei die Fällung bei Temperaturen von 65°C bis 100°C vorgenommen wird.

21. Verfahren nach einem der Ansprüche 12 bis 20, wobei nach Erreichen des End-pH-Wertes die erhaltene Fällsuspension für 0,5 bis 4 Stunden nachgerührt wird, bevor eine Weiterbearbeitung erfolgt.

22. Verfahren nach einem der Ansprüche 12 bis 21, wobei der basischen Lösung eine lösliche Si⁴⁺-Verbindung zugesetzt wird.

23. Verfahren nach Anspruch 22, wobei die lösliche Si⁴⁺-Verbindung Wasserglas ist.

## Claims

1. Catalyst, particularly for the hydrogenation of functional groups of organic compounds, containing nickel and/or cobalt on a TiO₂-containing carrier **characterized in that** the supported catalyst is produced through precipitation from a solution containing catalyst metal- and Ti⁴⁺-salts, is reduced, comprises metal crystallites with crystals sizes of <120 A, has a total content of nickel, cobalt or nickel and cobalt from 20 to 80 mass percent (based on the total mass of the catalyst) in the reduced and passivated state, has a TiO₂-content of from 10 to 80 mass percent, and has a reduction rate of at least 55% following a one-hour follow-up reduction at 100 °C.

2. Catalyst according to claim 1, wherein the TiO₂-containing carrier exhibits a SiO₂ -content of a maximum of 20 mass percent (based on the total mass of the catalyst).

3. Catalyst according to claims 1 or 2, wherein the TiO₂-containing carrier exhibits a ZrO₂ -content of a maximum of 60 mass percent (based on the total mass of the catalyst).

4. Catalyst according to any one of the claims 1 to 3, wherein the TiO₂-containing carrier comprises a HfO₂ -content of a maximum of 5 mass percent (based on the total mass of the catalyst).

5. Catalyst according to claim 1, wherein the reduced catalyst is stabilized.

6. Catalyst according to any one of the preceding claims, wherein the catalyst has a total metal content of cobalt and/or nickel of >50 mass percent and an average metal crystallite size of <100 Å.

7. Catalyst according to any one of the claims 1 to 5, wherein the catalyst has a metal content of cobalt and nickel of <50 mass percent and an average metal crystallite size of <60 A.

8. Catalyst according to any one of the claims 2 to 4, wherein the catalyst is present in reduced and stabilized form as a powder with grain sizes of from 1 to 200 µm.

9. Catalyst according to claim 5, wherein the catalyst is present as a powder with grain sizes from 1 to 30 µm.

10. Catalyst according to any one of the claims 1 to 8, wherein the catalyst is present in the form of extrudates, spheres or tablets.

11. Catalyst according to any one of the preceding claims, wherein the catalyst is present in a suitable medium selected from a group consisting of water, alcohols, fatty amines or other protective agents.

12. Process for the production of a nickel- and/or cobalt-containing catalyst on a carrier, particularly for the hydrogenation of functional groups of organic compounds, more particularly according to any one of the claims 1 to 11, wherein through precipitation from a solution containing a mixture of catalyst metal- and Ti⁴⁺-salts selected from a group consisting of a) Ni²⁺ and Ti⁴⁺, b) Ni²⁺ and Co²⁺ and Ti⁴⁺, and c) Co²⁺ and Ti⁴⁺ with a basic solution up to a pH from 7 to 9.5 a precipitation product will be obtained, which after filtration is washed, then dried at temperatures ranging from 80 to 150 °C, calcinated at temperatures ranging from 250 to 700 °C, and reduced with hydrogen at temperatures ranging from 300 to 550 °C, wherein at least 20% of the TiO₂₋ containing carrier in the reduced catalyst through the mutual precipitation from the solution containing the mixture of salts are obtained.

13. Process according to claim 12, wherein the basic solution is a solution of NaOH, NaHCO₃ or Na₂CO₃ or a mixture of at least two of these substances.

14. Process according to the claims 12 or 13, wherein the catalyst is made inert following the calcination.

15. Process according to any one of the claims 12 to 14, wherein the catalyst is made inert following the reduction.

16. Process according to any one of the claims 14 or 15, wherein the catalyst that has been reduced and optionally made inert is stabilized following the reduction or inertization, respectively.

17. Process according to any one of the claims 14 to 16, wherein the catalyst following reduction and optional inertization is directly introduced into a medium.

18. Process according to any one of the claims 12 to 17, wherein the solution containing a mixture of salts contains Zr⁴⁺.

19. Process according to any one of the claims 12 to 18, wherein the solution containing a mixture of salts contains portions of TiO₂ or TiO₂ -ZrO₂ mixtures or TiO₂-ZrO₂-SiO₂ mixtures, preferably in suspended form, wherein the portion of the TiO2-containing carrier in the reduced catalyst of the oxidic additives accounts for a maximum of 80%.

20. Process according to any one of the claims 12 to 19, wherein the precipitation is carried out at temperatures ranging from 65 °C to 100 °C.

21. Process according to any one of the claims 12 to 20, wherein after reaching the final pH the obtained precipitate suspension is stirred for 0.5 to 4 hours prior to further processing.

22. Process according to any one of the claims 12 to 21, wherein to the basic solution is added a soluble Si⁴⁺ compound.

23. Process according to claim 22, wherein the soluble Si⁴⁺ compound is water glass.

## Revendications

1. Catalyseur, en particulier pour l'hydrogénation de groupes fonctionnels de composés organiques, contenant du nickel et/ou du cobalt sur un support contenant du TiO₂, **caractérisé en ce que** le catalyseur mis sur support est fabriqué par précipitation à partir d'une solution contenant des sels de métal de catalyseur et de Ti⁴⁺, est réduit, présente des cristallites métalliques présentant des tailles de cristaux < 120 Å, possède à l'état réduit et passivé une teneur totale de 20 à 80 % en masse (sur la base de la masse totale du catalyseur) de nickel, de cobalt ou de nickel et de cobalt, possède une teneur en TiO₂ de 10 à 80 % en masse et possède, après une post-réduction d'une heure à 100 °C, un degré de réduction au moins égal à 55 %.

2. Catalyseur selon la revendication 1, dans lequel le support contenant du TiO₂ présente une teneur en SiO₂ au maximum égale à 20 % en masse (sur la base de la masse totale du catalyseur).

3. Catalyseur selon la revendication 1 ou 2, dans lequel le support contenant du TiO₂ présente une teneur en ZrO₂ au maximum égale à 60 % en masse (sur la base de la masse totale du catalyseur).

4. Catalyseur selon l'une des revendications 1 à 3, dans lequel le support contenant du TiO₂ présente une teneur en HfO₂ au maximum égale à 5 % en masse (sur la base de la masse totale du catalyseur).

5. Catalyseur selon la revendication 1, dans lequel le catalyseur réduit est rendu stable.

6. Catalyseur selon l'une des revendications précédentes, dans lequel le catalyseur présente une teneur totale en métal de cobalt et/ou de nickel > 50 % en masse et une taille moyenne des cristallites métalliques < 100 Å.

7. Catalyseur selon l'une des revendications 1 à 5, dans lequel le catalyseur présente une teneur en métal de cobalt et de nickel < 50 % en masse et une taille moyenne des cristallites métalliques < 60 Å.

8. Catalyseur selon l'une des revendications 2 à 4, dans lequel le catalyseur se présente à l'état réduit et stabilisé sous la forme d'une poudre présentant une grosseur de grains de 1 à 200 µm.

9. Catalyseur selon la revendication 5, dans lequel le catalyseur se présente sous la forme d'une poudre présentant une grosseur de grains comprise entre 1 et 30 µm.

10. Catalyseur selon l'une des revendications 1 à 8, dans lequel le catalyseur se présente sous la forme d'extrudats ou produits d'extrusion, de billes ou de comprimés.

11. Catalyseur selon l'une des revendications précédentes, dans lequel le catalyseur se présente dans un milieu approprié sélectionné parmi le groupe composé de l'eau, d'alcools, d'amines grasses ou d'autres agents protecteurs.

12. Procédé de fabrication d'un catalyseur contenant du nickel et/ou du cobalt sur un support, en particulier pour l'hydrogénation de groupes fonctionnels de composés organiques, en particulier selon l'une des revendications 1 à 11, dans lequel, par précipitation depuis une solution contenant un mélange de sels de métal de catalyseur et de Ti⁴⁺, sélectionnés parmi le groupe composé de a) Ni²⁺ et Ti⁴⁺, b) Ni²⁺ et Co²⁺ et Ti⁴⁺, et c) Co²⁺ et Ti⁴⁺, avec une solution basique jusqu'à une valeur de pH entre 7 et 9,5, on obtient un produit de précipitation qui est lavé après filtration, puis séché à des températures de 80°C à 150 °C, calciné à des températures de 250 à 700 °C, et réduit avec de l'hydrogène à des températures de 300 °C à 550 °C, où au moins 20 % du support contenant du TiO₂ dans le catalyseur réduit sont obtenus par la précipitation globale à partir de la solution contenant le mélange de sels.

13. Procédé selon la revendication 12, dans lequel la solution basique est une solution de NaOH, NaHCO₃ ou Na₂CO₃, ou un mélange d'au moins deux de ces substances.

14. Procédé selon la revendication 12 ou 13, dans lequel le catalyseur est rendu inerte après la calcination.

15. Procédé selon l'une des revendications 12 à 14, dans lequel le catalyseur est rendu inerte après la réduction.

16. Procédé selon l'une des revendications 14 ou 15, dans lequel le catalyseur réduit et éventuellement rendu inerte est stabilisé suite à la réduction ou à l'inertisation.

17. Procédé selon l'une des revendications 14 à 16, dans lequel le catalyseur, après la réduction et éventuellement l'inertisation, est directement incorporé dans un milieu.

18. Procédé selon l'une des revendications 12 à 17, dans lequel la solution contenant un mélange de sels contient du Zr⁴⁺.

19. Procédé selon l'une des revendications 12 à 18, dans lequel la solution contenant un mélange de sels contient des fractions de TiO₂ ou de mélanges TiO₂-ZrO₂ ou de mélanges TiO₂-ZrO₂-SiO₂, de préférence sous la forme d'une suspension, la fraction du support contenant du TiO₂ dans le catalyseur réduit provenant des additifs oxydants représentant au maximum 80 %.

20. Procédé selon l'une des revendications 12 à 19, dans lequel la précipitation est réalisée à des températures de 65 °C à 100 °C.

21. Procédé selon l'une des revendications 12 à 20, dans lequel, après l'obtention de la valeur de pH finale, la suspension précipitée obtenue est agitée encore pendant 0,5 à 4 heures, avant de subir un autre traitement.

22. Procédé selon l'une des revendications 12 à 21, dans lequel on ajoute à la solution basique un composé de Si⁴⁺ soluble.

23. Procédé selon la revendication 22, dans lequel le composé de Si⁴⁺ soluble est du verre soluble.
